(19) Europäisches Patentamt
European Patent Office
Office européen des brevets

(11) Veröffentlichungsnummer: **0 005 449**
**B1**

# (12) EUROPÄISCHE PATENTSCHRIFT

(45) Veröffentlichungstag der Patentschrift: **06.01.82**

(51) Int. Cl.[3]: **C 09 B 29/32**, C 09 D 11/16, C 07 D 277/66

(21) Anmeldenummer: **79101088.7**

(22) Anmeldetag: **09.04.79**

(54) **Wasserlösliche Monoazofarbstoffe, ihre Herstellung, konzentrierte wässrige Lösungen, welche die Monoazofarbstoffe enthalten, die Herstellung der Lösungen, die Verwendung der Farbstoffe und konzentrierten Lösungen und damit gefärbte und bedruckte Materialien.**

(30) Priorität: **16.05.78 DE 2821350**

(43) Veröffentlichungstag der Anmeldung: **28.11.79 Patentblatt 79/24**

(45) Bekanntmachung des Hinweises auf die Patenterteilung: **06.01.82 Patentblatt 82/1**

(84) Benannte Vertragsstaaten: **BE CH DE FR GB**

(56) Entgegenhaltungen:
**DE-A-1 932 246**
**GB-A-310 354**
**US-A-2 657 202**
**US-A-3 274 171**

(73) Patentinhaber: **CASSELLA Aktiengesellschaft, Hanauer Landstrasse 526, D-6000 Frankfurt am Main 61 (DE)**

(72) Erfinder: **Bauer, Wolfgang, Dr., Masurenstrasse 6, D-6457 Maintal 3 (DE)**
Erfinder: **Ribka, Joachim, Dr., Rügener Strasse 4, D-6050 Offenbach-Bürgel (DE)**

(74) Vertreter: **Urbach, Hans-Georg, Dr., Hanauer Landstrasse 526, D-6000 Frankfurt am Main 61 (DE)**

**EP 0 005 449 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Wasserlösliche Monoazofarbstoffe, ihre Herstellung, konzentrierte wäßrige Lösungen, welche die Monoazofarbstoffe enthalten, die Herstellung der Lösungen, die Verwendung der Farbstoffe und konzentrierten Lösungen und damit gefärbte und bedruckte Materialien

Die vorliegende Erfindung betrifft wasserlösliche Monoazofarbstoffe, die in Form der freien Säure der Formel I

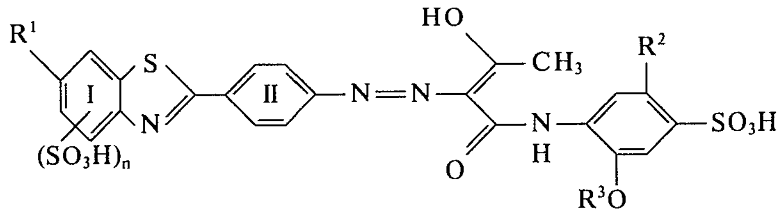

(I)

oder einer tautomeren Form derselben entsprechen, worin

$R^1$ = Methyl oder Äthyl oder 6-Methyl-7-sulfobenzthiazolyl-(2),
$R^2$ = Methyl, Methoxy oder Äthoxy,
$R^3$ = Methyl oder Äthyl und
n die Zahl 0 oder 1

bedeuten, wobei die Kerne I und/oder II durch eine weitere Methyl- oder Äthylgruppe substituiert sein können und die Sulfogruppen auch in Form ihrer Alkali- und/oder Ammoniumsalze vorliegen können, sowie ihre Herstellung, die Farbstoffe enthaltende konzentrierte wäßrige Lösungen, die Herstellung der Lösungen, die Verwendung der Farbstoffe und konzentrierten Lösungen und die damit gefärbten und bedruckten Materialien. Von den Alkalisalzen werden die Lithium- und Natriumsalze bevorzugt.

Im Rahmen der vorliegenden Erfindung werden Monoazofarbstoffe der allgemeinen Formel I bevorzugt, in denen $R^1$ = Methyl oder 6-Methyl-7-sulfobenzthiazolyl-(2) bedeutet und die Kerne I und/oder II durch weiteres Methyl substituiert sein können und die Sulfogruppen auch in Form ihrer Lithium-, Natrium- und/oder Ammoniumsalze vorliegen können.

Besonders bevorzugt sind Monoazofarbstoffe der Formel I, worin $R^1$ = Methyl oder 6-Methyl-7-sulfobenzthiazolyl-(2) bedeutet und die Kerne I und II keine weiteren Substituenten tragen und im Farbstoffmolekül zwei Sulfogruppen, die auch in Form ihrer Lithium-, Natrium- und/oder Ammoniumsalze vorliegen können, vorhanden sind.

Die erfindungsgemäßen Farbstoffe der Formel I können durch Diazotierung von Diazokomponenten der Formel II

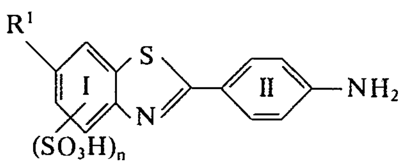

(II)

und Kupplung mit Kupplungskomponenten der Formel III

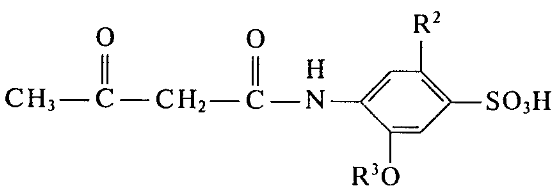

(III)

hergestellt werden, wobei $R^1$, $R^2$, $R^3$ und n in den Formeln II und III die obengenannte Bedeutung haben. Die Kupplung wird normalerweise im pH-Bereich von 2 bis 12, vorzugsweise 3 bis 9, und bei Temperaturen von $-10°C$ bis $+50°C$, vorzugsweise 0 bis 30°C, durchgeführt, wobei als Reaktionsmedium Wasser bevorzugt ist. Im wäßrigen Reaktionsmedium können jedoch gegebenenfalls einwertige oder mehrwertige Alkohole, beispielsweise Methanol, Äthanol, Isopropanol, n-Propanol, Äthylenglykol, Diäthylenglykol, Triäthylenglykol, Polyäthylenglykol, Glycerin, Pentaerythrit, Mannit, Sorbit, 1,2-Propylenglykol, 1,3-Propylenglykol, 1,3-, 2,3- und 1,4-Butylenglykol, Polypropylenglykol, Polyvinylalkohol, 1,6-Hexylenglykol, Äthylenglykolalkyläther, Diäthylenglykolalkyläther, Triäthylenglykolalkyläther, Polyäthylenglykolalkyläther, jeweils insbesondere mit 1 bis 4 C-Atomen im Alkylrest, oder amidische Verbindungen, beispielsweise Formamid, Dimethylformamid, ε-Caprolactam, Pyrrolidon, N-Alkylpyrrolidone mit 1 bis 4 C-Atomen im Alkylrest, Harnstoff, Thioharnstoff oder Amine, beispielsweise Pyridin, Äthanolamin, Diäthanolamin, Triäthanolamin, zur

Beschleunigung der Diazotierungs- und/oder Kupplungsreaktionen zugegen sein.

Die erfindungsgemäßen Monoazofarbstoffe der allgemeinen Formel I eignen sich hervorragend zum Färben und Bedrucken von hydroxylgruppenhaltigen oder stickstoffhaltigen natürlichen oder synthetischen Materialien, insbesondere von textilen Fasermaterialien, wie Baumwolle, Regeneratcellulose, Polyamid, Seide, Wolle sowie von Papier, Holz, Leder und als Schreibmittel.

Das Färben und Bedrucken erfolgt nach den üblichen Verfahren. Auf den genannten textilen Substraten erhält man brillante gelbe Färbungen bzw. Drucke guter Egalität, die sich vor allem durch eine überraschend hohe Farbstärke und Lichtechtheit und durch weitere gute Echtheitseigenschaften, insbesondere durch gute Wasch- und Naßechtheiten sowie Schweißechtheiten auszeichnen. Die Färbungen bzw. Drucke sind sehr gut ätzbar. Aufgrund der überraschend hohen Löslichkeit eignen sich die erfindungsgemäßen Monoazofarbstoffe insbesondere für halbkontinuierliche und kontinuierliche Färbeverfahren, beispielsweise für das pad-jig-, pad-roll-, pad-steam- und das Kaltverweil-Verfahren. Ferner weisen Färbeflotten aus den erfindungsgemäßen Farbstoffen eine geringe Elektrolytempfindlichkeit auf. Aufgrund des gleichmäßigen Ziehvermögens bei unterschiedlichen Färbetemperaturen sind die erfindungsgemäßen Monoazofarbstoffe auch sehr gut für Kombinationsfärbungen geeignet. Die neuen Monoazofarbstoffe eignen sich wegen ihrer hervorragenden Kaltwasserlöslichkeit ferner zum Färben von ungeleimtem oder geleimtem Papier in der Masse oder nach dem Tauchverfahren. Die Papierfärbungen zeichnen sich neben sehr guter Wasserechtheit, Zylinderechtheit, Ausblutechtheit, Säureechtheit, Alkaliechtheit, auch durch Beständigkeit gegenüber Fruchtsäften, Milch sowie alkoholischen Getränken aus. Besonders vorteilhaft ist, daß die hohe Lichtechtheit und hohe Farbstärke der erfindungsgemäßen Farbstoffe mit hoher Substantivität verbunden sind, so daß beim Färben von Baumwolle, Regeneratcellulose, Polyamid, Seide, Wolle, Leder oder bei der Papierherstellung ökologisch günstige Färbereiabwässer anfallen. Gegenüber vergleichbaren, bereits bekannten Monoazofarbstoffen, wie sie z. B. aus der USP 3 274 171 und der DOS 1 932 246 bekannt sind, weisen die erfindungsgemäßen Monoazofarbstoffe überraschende technische Vorteile, vor allem in der Lichtechtheit, der Farbstärke, der Substantivität, in den Naß- und Schweißechtheiten und in der Löslichkeit auf.

Als überraschend kann insbesondere die hohe Lichtechtheit der erfindungsgemäßen Farbstoffe gelten, weil es aus »Ulmanns Enzyklopädie der Technischen Chemie«, 3. Auflage, Band 4 (1953), Seite 105 bekannt war, daß von den Thiazol-Derivaten nur 2-(4'-Amino-3'-sulfo-phenyl)-6-methyl-benzthiazol, bei dem benachbart zu der diazotierbaren Aminogruppe eine Sulfogruppe enthalten ist, zu lichtechten Farbstoffen führt. Gegenüber derartigen Monoazofarbstoffen, wie sie beispielsweise in der deutschen Patentschrift 293 333 und in den US-Patentschriften 1 159 386, 2 657 202 beschrieben sind, weisen die erfindungsgemäßen Farbstoffe nicht nur eine deutlich bessere Farbstärke und Substantivität z. B. auf Polyamid und auf Leder auf, sondern besitzen zudem auch deutlich bessere Heiß- und Kaltwasserlöslichkeiten. Konzentrierte Färbeflotten, wie sie bei halbkontinuierlichen und kontinuierlichen Färbeverfahren verwendet werden, neigen daher bei den erfindungsgemäßen Farbstoffen nicht zum Auskristallisieren oder Gelieren und weisen außerdem eine erheblich geringere Elektrolytempfindlichkeit als die vergleichbaren bekannten Farbstoffe auf. Ein weiterer technischer Vorteil besteht darin, daß man die technisch leichter zugängliche und preiswertere 2-(4'-Aminophenyl)-6-methyl-benzthiazol-7-sulfonsäure anstatt der nach einem mehrstufigen Prozeß herstellbaren 2-(4'-Aminophenyl)-6-methyl-benzthiazol-3',7-disulfonsäure bei der Herstellung lichtechter Gelbfarbstoffe einsetzen kann. Ferner sind bei den erfindungsgemäßen Farbstoffen die Wasch- und Schweißechtheiten, z. B. auf Baumwolle oder Polyamid, besser.

Aus der GB-PS 310 354 sind Monoazofarbstoffe bekannt, die durch Diazotierung von 2-(4'-Amino-3'-sulfophenyl)-6-methylbenzthiazol (Dehydrothiotoluidin-ortho-monosulfonsäure) und anschließendes Kuppeln, z. B. auf Acetoacet-ortho-anisididsulfonsäure, hergestellt werden. Gegenüber diesen bekannten Farbstoffen besitzen die erfindungsgemäßen Farbstoffe eine bessere Löslichkeit, beim Färben auf Polyamid eine höhere Farbstärke sowie eine bessere Licht- und Schweißechtheit.

Die erfindungsgemäßen Monoazofarbstoffe können in Pulver- oder Granulatform, jedoch aufgrund ihrer hervorragenden Wasserlöslichkeit auch in Form konzentrierter wäßriger Lösungen zum Färben der genannten textilen oder nichttextilen natürlichen oder synthetischen Fasermaterialien eingesetzt werden. Die konzentrierten wäßrigen Lösungen enthalten dabei normalerweise 10 bis 30 Gew.-%, vorzugsweise 10 bis 20 Gew.-% Reinfarbstoff.

Zur Einstellung konzentrierter, lagerstabiler Lösungen werden an sich bekannte hydrotrope Verbindungen, beispielsweise aus der Reihe der hydrotropen Salze, wie Natriumbenzoat, benzolsulfonsaures Natrium, p-toluolsulfonsaures Natrium, xylolsulfonsaures Natrium, N-benzylsulfanilsaures Natrium, oder der amidischen, carbonylgruppenhaltigen Verbindungen, beispielsweise Formamid, Dimethylformamid, Acetamid, ε-Caprolactam, N-Methylpyrrolidon, Harnstoff, Thioharnstoff, oder der Alkohole, beispielsweise Äthanol, n-Propanol, Isopropanol, Äthylenglykol, Diäthylenglykol, Triäthylenglykol, Polyäthylenglykol, Polypropylenglykol, 1,2-Propylenglykol, 1,3-Propylenglykol, 1,3-Butylenglykol, 2,3-Butylenglykol, 1,4-Butylenglykol, 2,2-Diäthyl-propandiol-(1,3), 1,6-Hexylenglykol, 3-Methyl- und 2-Methyl-hexylenglykol-(1,6), 2-Methylpentandiol-(2,4), 2-Äthyl-hexandiol-(1,3), 2,2-Dimethylhexandiol-(1,3), Diäthylenglykolalkyläther, Äthylenglykolalkyläther, Triäthy-

lenglykolalkyläther, Polyäthylenglykolalkyläther, jeweils insbesondere mit 1 bis 4 C-Atomen im Alkylrest, oder der Amine, wie Pyridin, Monoäthanolamin, Diäthanolamin, Triäthanolamin eingesetzt. Hydrotrope Verbindungen sind beispielsweise beschrieben in H. Rath und S. Müller, Melliand Textilberichte 40 (1959), 787 oder bei E. H. Daruwalla, K. Venkataraman: »The Chemistry of Synthetic Dyes«, Vol. VII, Seiten 86 bis 92 (1974) und in der dort zitierten Literatur. Bevorzugte hydrotrope Mittel, die sich zur Einstellung lagerstabiler, konzentrierter Lösungen der erfindungsgemäßen Farbstoffe eignen, sind amidische Verbindungen, insbesondere Harnstoff und/oder ε-Caprolactam und mehrwertige Alkohole, insbesondere Alkylenglykole mit 2 bis 6 C-Atomen in der Alkylenkette oder Gemische dieser Verbindungen. Normalerweise enthalten die konzentrierten, lagerstabilen Lösungen 5 bis 40 Gew.-%, vorzugsweise 10 bis 30 Gew.-%, einer oder mehrerer hydrotroper Verbindungen.

In den konzentrierten Lösungen bzw. den Pulver- oder Granulatformen der erfindungsgemäßen Farbstoffe können an sich bekannte Hilfsmittel, wie z. B. Tenside, die sich zur besseren Benetzbarkeit und/oder Löslichkeitsverbesserung von Direktfarbstoffen eignen, zugegen sein. Als Tenside kommen Aniontenside, Amphotenside oder nichtionogene Tenside in Betracht, wie sie z. B. beschrieben sind in a) Ullmanns Enzyklopädie der technischen Chemie, 3. Auflage, Band 16 (1965), Seiten 724 bis 748; b) J. L. Moillet, B. Collie und W. Black in »Surface Activity«, 2. Auflage, Kap. 10 bis 15; c) E. H. Daruwalla in K. Venkataraman »The Chemistry of Synthetic Dyes«, Vol. VII (1974), Seiten 86 bis 92.

Bei der Herstellung von konzentrierten lagerstabilen wäßrigen Lösungen der erfindungsgemäßen Farbstoffe ist die Isolierung der Farbstoffpreßkuchen bzw. Farbstoffpulver und ihre anschließende Auflösung in Wasser nicht unbedingt erforderlich. Konzentrierte wäßrige Lösungen können auch dadurch erhalten werden, daß man eine oder mehrere Diazokomponenten der Formel II zunächst diazotiert und dann mit einer oder mehrerer Kupplungskomponenten der Formel III vereinigt oder daß man ein wäßriges Gemisch einer Diazokomponente der Formel II mit einer Kupplungskomponente der Formel III mit Alkalinitrit umsetzt. Dabei kann man auch ein Diazokomponenten- und/oder Kupplungskomponentengemisch einsetzen. Diazokomponente, Kupplungskomponente und Alkalinitrit werden dabei etwa im Molverhältnis 1 : 1 : 1 eingesetzt. Der Zusatz einer zusätzlichen Säure ist nicht erforderlich, jedoch ist die Zugabe von Eis zur Kühlung zweckmäßig. Die Reaktionstemperaturen betragen normalerweise 0 bis 30° C, vorzugsweise 15 bis 25° C.

Bei diesen Verfahren müssen an sich bekannte hydrotrope Verbindungen und/oder Tenside, beispielsweise der bereits genannten Art, während der Diazotierungs- und/oder Kupplungsreaktionen oder nach erfolgter Kupplung zugesetzt werden. Bevorzugte hydrotrope Mittel zur Herstellung konzentrierter Lösungen nach diesen Verfahren stammen aus der Reihe der amidischen hydrotropen Mittel, insbesondere Harnstoff und/oder ε-Caprolactam, und der mehrwertigen Alkohole, insbesondere Alkylenglykole mit 2 bis 6 C-Atomen im Alkylenrest oder Gemische dieser Verbindungen. Auch nach den zuletzt genannten Verfahren lassen sich konzentrierte lagerstabile wäßrige Farbstofflösungen herstellen, die etwa 10 bis 30 Gew.-%, vorzugsweise 10 bis 20 Gew.-%, Reinfarbstoff und 5 bis 40 Gew.-%, vorzugsweise 10 bis 30 Gew.-%, einer oder mehrerer hydrotropen Verbindungen enthalten.

Als Diazokomponenten können zur Herstellung der erfindungsgemäßen Monoazofarbstoffe beispielsweise eingesetzt werden:

2-(4'-Aminophenyl)-6-methylbenzthiazol (Dehydrothiotoluidin),
2-(4'-Aminophenyl)-6-methyl-benzthiazol-7-sulfosäure (Dehydrothiotoluidin-monosulfosäure),
2-(4'-Aminophenyl)-6-methyl-benzthiazol-5-sulfonsäure,
2-(4'-Aminophenyl)-6-(6''-methyl-benzthiazolyl(2''))-benzthiazol-7''-monosulfosäure (Primulinsäure),
2-(3'-Methyl-4'-aminophenyl)-4,6-dimethyl-benzthiazol (Dehydrothio-m-xylidin),
2-(3'-Methyl-4'-aminophenyl)-4,6-dimethyl-benzthiazol-7-sulfonsäure (Dehydrothio-m-xylidin-monosulfonsäure) oder
Gemische dieser Diazokomponenten.

Als Kupplungskomponenten können zur Herstellung der erfindungsgemäßen Monoazofarbstoffe der Formel I beispielsweise eingesetzt werden:

1-Acetoacetyl-amino-2-methoxy-5-methyl-benzol-4-sulfonsäure,
1-Acetoacetylamino-2-äthoxy-5-methyl-benzol-4-sulfonsäure,
1-Acetoacetylamino-2,5-dimethoxy-benzol-4-sulfonsäure,
1-Acetoacetylamino-2,5-diäthoxy-benzol-4-sulfonsäure oder
Gemische dieser Kupplungskomponenten.

In den folgenden Beispielen bedeuten Teile Gewichtsteile, Prozente Gewichtsprozente, die Temperaturen sind in Celsiusgraden angegeben.

## Beispiel 1

32 Teile 2-(4'-Aminophenyl)-6-methylbenzthiazol-7-sulfonsäure (Dehydrothiotoluidin-monosulfosäure) werden in 200 Teile Wasser eingetragen, mit einer Lösung von 40 Teilen Natriumhydroxid in 100 Teilen Wasser neutral gelöst und mit einer Lösung von 7,59 Teilen Natriumnitrit in 20 Teilen Wasser

versetzt. Die erhaltene Mischung läßt man anschließend langsam zu einem gut gerührten Gemisch aus 26 Teilen 32%iger wäßriger Salzsäure, 200 Teilen Eis und 100 Teilen Wasser zulaufen. Man läßt ca. 1 Stunde bei 0 bis 10°C nachrühren und entfernt dann überschüssige salpetrige Säure mit Amidosulfonsäure. Die Suspension der Diazoverbindung läßt man anschließend zu einem Gemisch von 33,4 Teilen 1-acetoacetylamino-2-methoxy-5-methylbenzol-4-sulfonsaurem Ammonium, 15,6 Teilen Natriumhydrogencarbonat, 100 Teilen Eis und 100 Teilen Wasser zulaufen. Zur Vervollständigung der Kupplungsreaktion rührt man mehrere Stunden bei pH 6 bis 7 nach und isoliert den Farbstoff der Formel

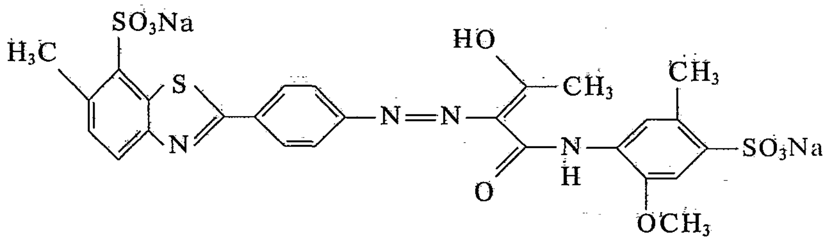

(IV)

durch Zusatz von Natriumchlorid. Man erhält 200 Teile einer ca. 33%igen gelben Farbstoffpaste, die nach Trocknung (Schrank- oder Sprühtrocknung) 90 Teile eines 72%igen gelben Pulvers (96% der Theorie) mit hoher Wasserlöslichkeit liefert.

Beispiel 2

45,3 Teile 2-(4′-Aminophenyl)-6-(6″-methyl-benzthiazolyl-(2″))-benzthiazol-7″-monosulfonsäure (Primulinsäure) werden nach den Angaben des Beispiels 1 diazotiert und mit 33,4 Teilen 1-acetoacetylamino-2-methoxy-5-methylbenzol-4-sulfonsaurem Ammonium bei pH 6 bis 7 gekuppelt. Der mit Hilfe von Natriumchlorid abgeschiedene Farbstoff der Struktur

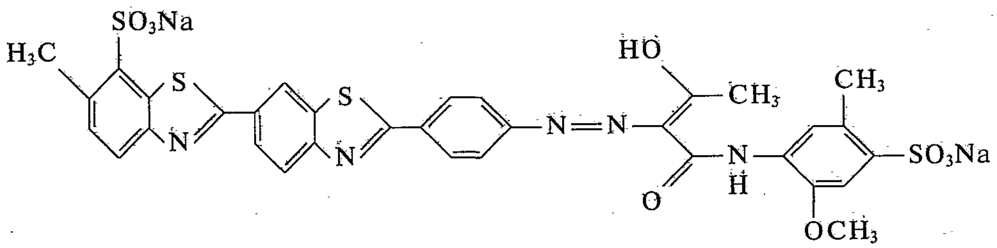

(V)

wird abfiltriert. Man erhält 260 g eines ca. 40%igen Farbstoffpreßkuchens, der nach Schrank- oder Sprühtrocknung 105 g (90% der theoretischen Ausbeute) eines gelben Pulvers hoher Wasserlöslichkeit liefert.

Beispiel 3

32 Teile 2-(4′-Aminophenyl)-6-methylbenzthiazol-7-sulfosäure (Dehydrothiotoluidin-monosulfosäure) und 33,4 Teile 1-acetoacetylamino-2-methoxy-5-methylbenzol-4-sulfonsaures Ammonium werden in eine Mischung von 100 Teilen ε-Caprolactam und 160 Teilen Wasser eingetragen und mit einer Lösung von 7,1 Teilen Natriumnitrit in 10 Teilen Wasser sowie ca. 30 Teilen Eis versetzt. Man rührt ca. 2 Stunden bei einer Temperatur von 15 bis 25°C, wobei sich ein pH von 3,5 bis 4,5 einstellt und erhält ca. 380 Teile einer lagerbeständigen, ca. 17%igen färbefertigen Lösung des Farbstoffs der Formel

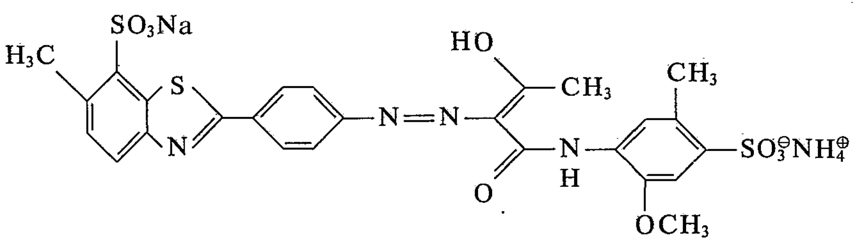

(VI)

Beispiel 4

Eine gut gerührte Mischung von 45,4 Teilen 2-(4'-Aminophenyl)-6-(6''-methyl-benzthiazolyl(2''))-benzthiazol-7''-sulfosäure (Primulinsäure), 33,4 Teilen 1-acetoacetylamino-2-methoxy-5-methyl-benzol-4-sulfonsaurem Ammonium, 100 Teilen Äthylenglykol in 170 Teilen Wasser werden unter gleichzeitiger Zugabe von ca. 40 Teilen Eis mit einer Lösung von 7,1 Teilen Natriumnitrit in 10 Teilen Wasser versetzt. Man rührt 2 bis 3 Stunden bei 15 bis 25° C, wobei sich ein pH-Wert von 3,5 bis 4,5 einstellt und erhält nach einer Klärfiltration ca. 400 Teile einer ca. 18%igen färbefertigen Lösung des Farbstoffs der Formel

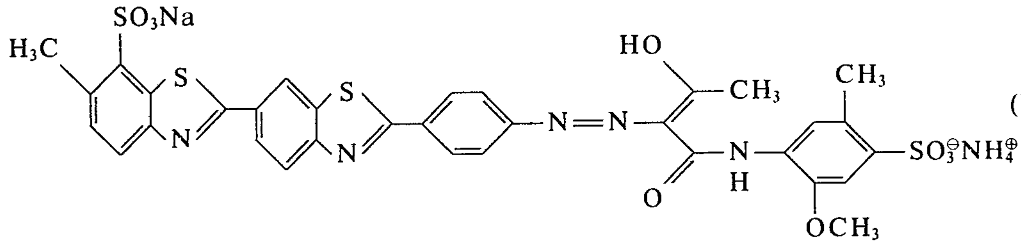

(VII)

Beispiel 5

Verfährt man nach den Angaben des Beispiels 4 unter Verwendung von 100 Teilen ε-Caprolactam anstelle von 100 Teilen Äthylenglykol, so erhält man ebenfalls ca. 400 Teile einer ca. 18%igen färbefertigen Lösung des Farbstoffs der Formel VII.

Beispiel 6

30 Teile der nach Beispiel 1 erhaltenen Farbstoffpaste werden in eine Lösung von 30 Teilen Harnstoff in 40 Teilen Wasser eingetragen und bei 30 bis 50° C gelöst. Man erhält so eine lagerbeständige Lösung des Monoazofarbstoffs der Formel IV.

Beispiel 7

Verfährt man nach den Angaben des Beispiels 6, setzt jedoch 30 Teile ε-Caprolactam anstatt 30 Teile Harnstoff ein, so erhält man ebenfalls eine lagerbeständige, echte Lösung des Monoazofarbstoffs der Formel IV.

Beispiel 8

30 Teile der nach Beispiel 1 erhaltenen Farbstoffpaste werden in eine Lösung von 30 Teilen Äthylenglykol in 40 Teilen Wasser eingerührt und bei ca. 50°C gelöst. Die erhaltene färbefertige Lösung des Farbstoffs der Formel IV ist lagerstabil.

Beispiel 9

26 Teile des nach Beispiel 2 gewonnenen Farbstoffpreßkuchens werden in eine Lösung von 30 Teilen Harnstoff in 40 Teilen Wasser eingerührt und bei ca. 50°C gelöst. Man erhält eine färbefertige, lagerbeständige Lösung des Farbstoffs der Formel V.

Beispiel 10

In einem Färbebecher, der sich in einem beheizbaren Bad befindet, stellt man bei 40° C eine Lösung von 4 Teilen Glaubersalz in 200 Teilen Wasser her. Man fügt 0,1 Teile des nach Beispiel 1 erhaltenen Farbstoffpulvers zu und hält in der fertigen Färbeflotte 10 g Baumwollgewebe ständig in Bewegung. Man erhöht die Temperatur des Färbebades auf 90°C und färbt 45 Minuten bei dieser Temperatur weiter. Das gefärbte Baumwollgewebe wird darauf der nur noch schwach gefärbten Restflotte entnommen und anhaftende Restflotte durch Auswringen entfernt. Anschließend wird das gefärbte Material mit kaltem Wasser gespült und bei 60°C getrocknet. Man erhält eine grünstichig gelbe, brillante Färbung hoher Farbstärke und sehr guter Egalität mit guten Echtheitseigenschaften,

insbesondere hoher Lichtechtheit, guten Wasch- und Schweißechtheiten, guter Alkali-, Säure- und Formaldehydechtheit, guter Ätzbarkeit und geringer Farbtonänderung bei der bei Direktfarbstoffen üblichen Hochveredlung.

### Beispiel 11

Färbt man 10 g Baumwollgewebe nach den Angaben des Beispiels 10 mit 0,1 Teilen des nach Beispiel 2 erhaltenen Farbstoffs der Formel V, so erhält man eine rotstichig gelbe Färbung guter Egalität und hoher Farbstärke, die sich neben guter Lichtechtheit durch gute Wasch-, Schweiß-, Säure-, Alkali- und Formaldehydechtheiten sowie durch geringe Farbtonänderung bei der Hochveredlung auszeichnet.

### Beispiel 12

0,15 Teile des nach Beispiel 1 erhaltenen Farbstoffpulvers und 2 Teile 10%ige wäßrige Ammoniumacetatlösung werden in einem Färbebecher mit Wasser auf 250 Teile aufgefüllt und in einem Färbeapparat auf 40°C erwärmt. In dieser Färbeflotte werden 10 g Polyamidgarn ständig in Bewegung gehalten, wobei 10 Minuten bei 40°C gefärbt wird und anschließend die Färbetemperatur im Verlauf von 30 Minuten auf 90°C erhöht wird. Man färbt 90 Minuten bei 90°C weiter, wobei nach jeweils 30 Minuten 1 ml einer 3%igen wäßrigen Essigsäurelösung zugesetzt wird. Anschließend wird das gefärbte Garn mit kaltem Wasser gespült und getrocknet. Man erhält eine brillante, grünstichig gelbe Färbung guter Egalität und hoher Farbstärke, die eine hohe Lichtechtheit sowie gute Wasch- und Schweißechtheiten aufweist.

### Beispiel 13

50 Teile 100% gebleichter Sulfitzellstoff in 1000 Teilen Wasser werden in einem Holländer gemahlen. Zu der fein verteilten Sulfitzellstoffmasse gibt man 0,5 Teile der nach Beispiel 3 erhaltenen konzentrierten Farbstofflösung und mischt ca. 15 Minuten weiter. Der Papierstoff kann geleimt werden. Anschließend erfolgt die Papierherstellung. Man erhält auf ungeleimten oder geleimten Papieren eine brillante, grünstichig gelbe Färbung hoher Farbstärke mit guten Echtheitseigenschaften, insbesondere guter Lichtechtheit, guter Ausblutechtheit, guter Säure- und Alkaliechtheit sowie guter Wasser- und Alkoholechtheit.

Das bei der Papiermassefärbung anfallende Abwasser ist ökologisch günstig.

Der folgenden Tabelle ist der strukturelle Aufbau weiterer Monoazofarbstoffe zu entnehmen, die entsprechend den Beispielen 1 bis 5 hergestellt werden können.

In der Tabelle ist angegeben:

zu Spalte 1 die benutzte Diazokomponente der Formel II,
zu Spalte 2 die benutzte Kupplungskomponente der Formel III,
zu Spalte 3 der Farbton der erfindungsgemäßen Monoazofarbstoffe auf Baumwolle.

| Beispiele | Diazokomponente der Formel (II) | Kupplungskomponente der Formel (III) | Farbton auf Baumwolle |
|---|---|---|---|
| 14 | 2-(4'-Aminophenyl)-6-methyl-benzthiazol (Dehydrothiotoluidin) | 1-Acetoacetylamino-2-methoxy-5-methylbenzol-4-sulfonsäure | gelb |
| 15 | 2-(3'-Methyl-4'-aminophenyl)-4,6-dimethyl-benzthiazol-7-sulfonsäure (Dehydrothio-m-xylidin-monosulfonsäure | 1-Acetoacetylamino-2-methoxy-5-methylbenzol-4-sulfonsäure | gelb |
| 16 | 2-(4'-Aminophenyl)-6-methyl-benzthiazol-7-sulfonsäure (Dehydrothiotoluidin-monosulfonsäure) | 1-Acetoacetylamino-2-äthoxy-5-methylbenzol-4-sulfonsäure | gelb |
| 17 | 2-(4'-Aminophenyl)-6-methyl-benzthiazol-7-sulfonsäure (Dehydrothiotoluidin-monosulfonsäure) | 1-Acetoacetylamino-2,5-dimethoxy-benzol-4-sulfonsäure | gelb |
| 18 | 2-(4'-Aminophenyl)-6-methyl-benzthiazol-7-sulfonsäure | 1-Acetoacetylamino-2,5-diäthoxy-benzol-4-sulfonsäure | gelb |
| 19 | 2-(4'-Aminophenyl)-6-methyl-benzthiazol-7-sulfonsäure/2-(4'-Amino-phenyl)-6-(6"-methyl-benzthiazolyl(2"))-benzthiazol-7"-sulfonsäure (Primulinsäure) Molverhältnis 1 : 1 | 1-Acetoacetylamino-2-methoxy-5-methylbenzol-4-sulfonsäure | gelb |
| 20 | Primulinsäure | 1-Acetoacetylamino-2,5-dimethoxy-benzol-4-sulfonsäure | gelb |
| 21 | 2-(4'-Aminophenyl)-6-methyl-benzthiazol-7-sulfonsäure | 1-Acetoacetylamino-2-methoxy-5-methylbenzol-4-sulfonsäure/ 1-Acetoacetylamino-2,5-dimethoxy-benzol-4-sulfonsäure (Molverhältnis 1 : 1) | gelb |

**Patentansprüche**

1. Wasserlösliche Monoazofarbstoffe der Formel I

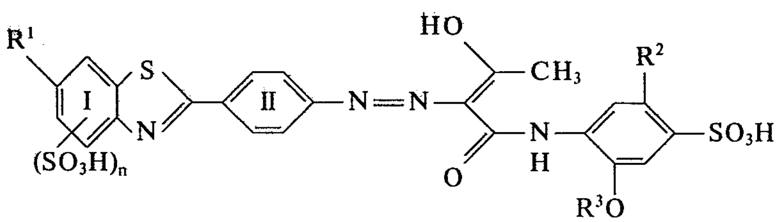

(I)

oder einer tautomeren Form derselben, worin

$R^1$ = Methyl, Äthyl oder 6-Methyl-7-sulfo-benzthiazolyl-(2),
$R^2$ = Methyl, Methoxy oder Äthoxy,
$R^3$ = Methyl oder Äthyl und
n = 0 oder 1

bedeuten, wobei die Kerne I und/oder II durch eine weitere Methyl- oder Äthylgruppe substituiert sein können und die Sulfogruppen auch in Form ihrer Alkali- und/oder Ammoniumsalze vorliegen können.

2. Monoazofarbstoffe nach Anspruch 1, dadurch gekennzeichnet, daß $R^1$ = Methyl oder 6-Methyl-7-sulfo-benzthiazolyl-(2) bedeutet und die Kerne I und/oder II durch eine weitere Methylgruppe substituiert sein können und die Sulfogruppen in Form ihrer Lithium-, Natrium- und/oder Ammoniumsalze vorliegen können.

3. Monoazofarbstoffe nach den Ansprüchen 1 oder 2, dadurch gekennzeichnet, daß $R^1$ = Methyl

oder 6-Methyl-7-sulfo-benzthiazolyl-(2) bedeutet und die Kerne I und/oder II keine weiteren Substituenten tragen und im Farbstoffmolekül zwei Sulfogruppen, die auch in Form ihrer Lithium-, Natrium- und/oder Ammoniumsalze vorliegen können, vorhanden sind.

4. Konzentrierte wäßrige Lösungen, enthaltend einen Monoazofarbstoff nach den Ansprüchen 1 bis 3.

5. Konzentrierte wäßrige Lösungen nach Anspruch 4, dadurch gekennzeichnet, daß sie eine oder mehrere hydrotrope Verbindungen enthalten.

6. Konzentrierte wäßrige Lösungen nach Anspruch 5, dadurch gekennzeichnet, daß sie als hydrotrope Mittel Harnstoff und/oder ε-Caprolactam und/oder Alkylenglykole mit 2 bis 6 C-Atomen im Alkylenrest und/oder Amide und/oder Alkohole enthalten.

7. Verfahren zur Herstellung wasserlöslicher Monoazofarbstoffe der Ansprüche 1 bis 3, dadurch gekennzeichnet, daß man eine Diazokomponente der Formel II

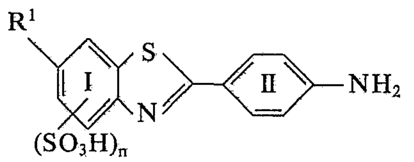

(II)

diazotiert und mit einer Kupplungskomponente der Formel III

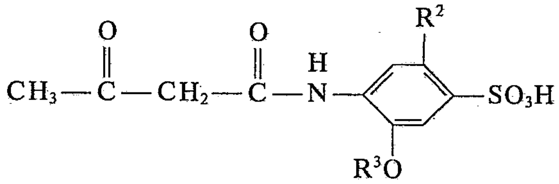

(III)

kuppelt, wobei $R^1$, $R^2$, $R^3$ und n die in den Ansprüchen 1 bis 3 wiedergegebenen Bedeutungen haben.

8. Verfahren zur Herstellung der konzentrierten wäßrigen Lösungen der Ansprüche 4 bis 5, dadurch gekennzeichnet, daß man ein wäßriges Gemisch einer Diazokomponente der Formel II

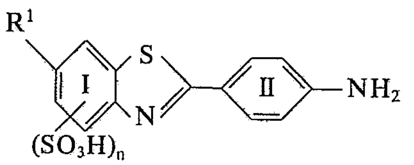

(II)

einer Kupplungskomponente der Formel III

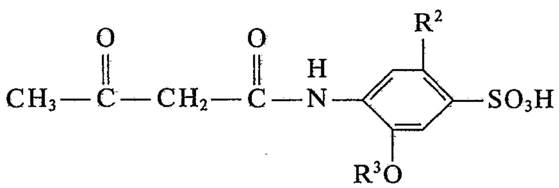

(III)

wobei $R^1$, $R^2$, $R^3$ und n die in den Ansprüchen 1 bis 3 wiedergegebenen Bedeutungen haben, und einer hydrotropen Verbindung aus der Reihe der Amide und/oder Alkohole mit einem Alkalinitrit versetzt.

9. Verwendung der wasserlöslichen Monoazofarbstoffe der Ansprüche 1 bis 3 in Form ihrer Farbstoffpulver oder Granulate oder in Form von konzentrierten wäßrigen Lösungen der Ansprüche 4 bis 6 zum Färben und Bedrucken von hydroxylgruppenhaltigen oder stickstoffhaltigen natürlichen oder synthetischen Materialien und als Schreibmittel.

10. Die mit den wasserlöslichen Monoazofarbstoffen der Ansprüche 1 bis 3 oder den konzentrierten wäßrigen Lösungen der Ansprüche 4 bis 6 gefärbten oder bedruckten hydroxylgruppenhaltigen oder stickstoffhaltigen natürlichen oder synthetischen Materialien.

**Claims**

1. Water-soluble monoazo dyestuffs of the formula I

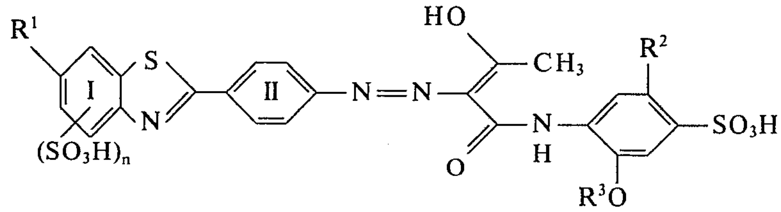

(I)

or a tautomeric form thereof, wherein

$R^1$ is methyl, ethyl or 6-methyl-7-sulphobenzthiazol-2-yl,
$R^2$ is methyl, methoxy or ethoxy,
$R^3$ is methyl or ethyl and
n is 0 or 1,

it being possible for the nuclei I and/or II to be substituted by a further methyl or ethyl group and for the sulpho groups to be present in the form of their alkali metal and/or ammonium salts.

2. Monoazo dyestuffs as claimed in claim 1, characterised in that $R^1$ is methyl or 6-methyl-7-sulphobenzthiazol-2-yl, and that the nuclei I and/or II may be substituted by a further methyl group and that the sulpho groups may be present in the form of their lithium, sodium and/or ammonium salts.

3. Monoazo dyestuffs as claimed in claim 1 or 2, characterised in that $R^1$ is methyl or 6-methyl-7-sulphobenzthiazol-2-yl and that the nuclei I and/or II carry no further substituents and that in the dyestuff molecule two sulpho groups are present which may also be in the form of their lithium, sodium and/or ammonium salts.

4. Concentrated aqueous solutions containing a monoazo dyestuff as claimed in claims 1 to 3.

5. Concentrated aqueous solutions as claimed in claim 4, characterised in that they contain one or several hydrotropic compounds.

6. Concentrated aqueous solutions as claimed in claim 5, characterised in that they contain as hydrotropic agents urea and/or ε-caprolactam and/or alkylene glycols having 2 to 6 carbon atoms in the alkylene moiety and/or amides and/or alcohols.

7. Method of producing water-soluble monoazo dyestuffs as claimed in claims 1 to 3, characterised in that a diazo component of the formula II

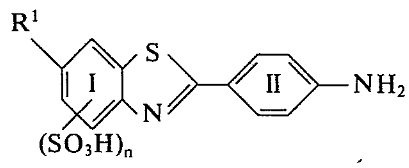

(II)

is diazotised and coupled with a coupling component of the formula III

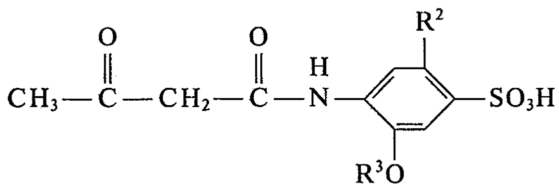

(III)

in which formulae $R^1$, $R^2$, $R^3$ and n have the meanings given in claims 1 to 3.

8. Method of producing the concentrated aqueous solutions as claimed in claims 4 and 5, characterised in that an alkali nitrite is added to a mixture composed of a diazo component of the formula II

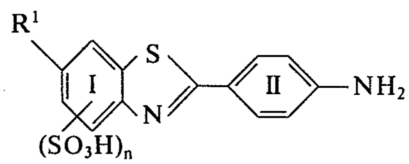

(II)

of a coupling component of the formula III

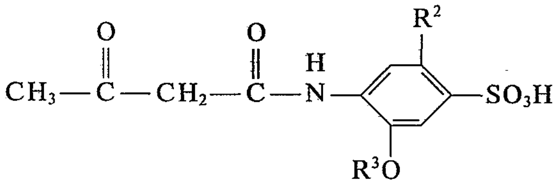

(III)

in which formulae $R^1$, $R^2$, $R^3$ and n have the meanings given in claims 1 to 3, and a hydrotropic compound selected from the series comprising amides and/or alcohols.

9. Use of the water-soluble monoazo dyestuffs as claimed in claims 1 to 3 in the form of their dyestuff powders or granules or in the form of concentrated aqueous solutions of claims 4 to 6 for dyeing and printing natural or synthetic materials containing hydroxyl groups or nitrogen, and as writing agents.

10. The natural or synthetic materials containing hydroxyl groups or nitrogen and dyed or printed with the water-soluble monoazo dyestuffs of claims 1 to 3 or the concentrated aqueous solutions of claims 4 to 6.

**Revendications**

1. Colorants monoazoïques hydrosulubles de formule I

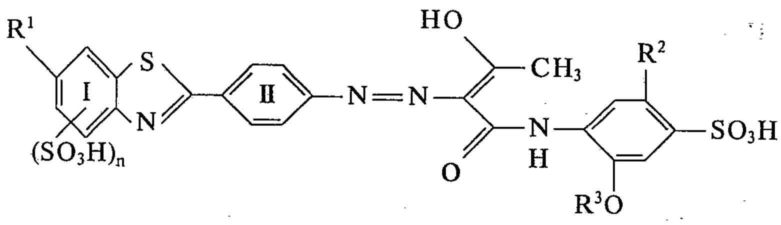

(I)

ou d'une forme tautomère de celle-ci, dans laquelle

$R^1$ représente un groupe méthyle, éthyle ou méthyl-6 sulfo-7 benzothiazolyle-2,
$R^2$ représente un groupe méthyle, méthoxy ou éthoxy,
$R^3$ représente un groupe méthyle ou éthyle et
n représente le nombre 0 ou 1,

les noyaux I et/ou II pouvant en outre porter encore un autre groupe méthyle ou éthyle, et les groupes sulfo pouvant également se trouver sous la forme de groupes sulfonates de métaux alcalins et/ou d'ammonium.

2. Colorants monoazoïques selon la revendication 1, caractérisés en ce que $R^1$ représente un groupe méthyle ou méthyl-6 sulfo-7 benzothiazolyle-2, les noyaux I et/ou II pouvant porter encore un autre groupe méthyle et les groupes sulfo pouvant également se trouver sous la forme de groupes sulfonates de lithium, de sodium et/ou d'ammonium.

3. Colorants monoazoïques selon la revendication 1 ou 2, caractérisés en ce que $R^1$ représente un groupe méthyle ou méthyl-6 sulfo-7 benzothiazolyle-2 et en ce que les noyaux I et/ou II ne portent pas d'autres substituants, la molécule contenant deux groupes sulfo pouvant encore se présenter sous forme de groupes sulfonates de lithium, sodium et/ou ammonium.

4. Solutions aqueuses concentrées, contenant un colorant monoazoïque selon les revendications 1 à 3.

5. Solutions aqueuses concentrées selon la revendication 4, caractérisées en ce qu'elles contiennent un ou plusieurs composés hydrotropes.

6. Solutions aqueuses concentrées selon la revendication 5, caractérisées en ce qu'elles contiennent, comme composés hydrotropes, de l'urée et/ou de l'ε-caprolactame et/ou des alkylèneglycols avec 2 à 6 atomes de carbone dans le radical alkylène et/ou des amides et/ou des alcools.

7. Procédé de préparation des colorants monoazoïques hydrosolubles des revendications 1 à 3, caractérisé en ce qu'on diazote une composante diazoïque de formule II

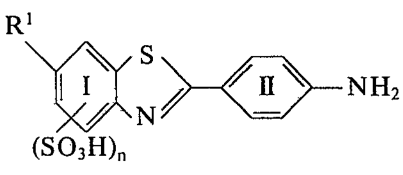

(II)

et on copule avec une composante de copulation de formule III,

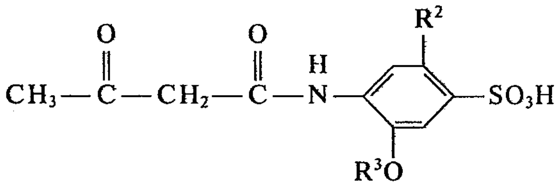

(III)

formules dans lesquelles $R^1$, $R^2$, $R^3$ et n ont les significations données dans les revendications 1 à 3.

8. Procédé de préparation des solutions aqueuses concentrées des revendications 4 à 5, caractérisé en qu'on fait réagir un mélange aqueux d'une composante diazoïque de formule II

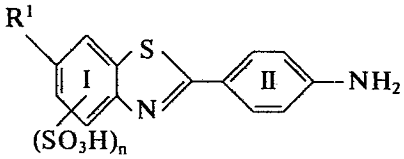

(II)

d'une composante de copulation de formule III

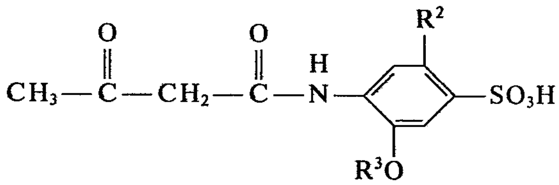

(III)

$R^1$, $R^2$, $R^3$ et n ayant les significations données dans les revendications 1 à 3, et d'un composé hydrotrope de la série des amides et/ou des alcools, avec un nitrite de métal alcalin.

9. Utilisation des colorants monoazoïques hydrosolubles des revendications 1 à 3 sous forme de poudres ou de granulés des colorants, ou sous forme de solutions aqueuses concentrées des revendications 4 à 6, pour la teinture et l'impression de matières naturelles ou synthétiques contenant des groupes hydroxyles ou de l'azote, ainsi que pour l'écriture.

10. Matières naturelles ou synthétiques contenant des groupes hydroxyles ou de l'azote, teintes ou imprimées à l'aide des colorants monoazoïques hydrosolubles des revendications 1 à 3 ou à l'aide des solutions aqueuses concentrées des revendications 4 à 6.